# EUROPEAN PATENT APPLICATION

(11) **EP 4 137 561 A1**
(43) Date of publication of application: **22.02.2023**
(21) Application number: 21779299.3
(22) Date of filing: 01.04.2021
(51) Int. Cl.: C12N 5/0775, A61K 35/51, A61K 9/00, A61P 17/02, A61K 8/98, A61Q 19/00

(54) **METHOD FOR PREPARING CULTURE MEDIUM CONTAINING HIGH LEVELS OF HIGH-POTENCY EXOSOMES SECRETED BY CORD BLOOD STEM CELLS, AND USE THEREOF**

(30) Priority: 01.04.2020 KR 20200039848
(71) Applicant: Primoris Co., Ltd., Gyeonggi-do 14322 (KR)
(72) Inventor: LEE, Dong-yeol, Seoul 06732 (KR); NA, Kuy-heum, Suwon-si Gyeonggi-do 16707 (KR); AHN, Hee-jin, Seoul 02598 (KR); LEE, Jae-yong, Gwangju-si Gyeonggi-do 12777 (KR); LEE, Yea-in, Gwangmyeong-si Gyeonggi-do 14316 (KR)
(74) Representative: ZBM Patents - Zea, Barlocci & Markvardsen
(86) International application number: PCT/KR2021/004088
(87) International publication number: WO 2021/201637

(57) **Abstract**

The present invention relates to a method for preparing a culture medium containing exosomes released by umbilical cord blood stem cells. The preparation method is based on culturing the umbilical cord blood stem cells in a serum-free medium supplemented with GDF-11, EGF, FGF2, TFG-β1, and/or VEGF. The culture medium produced by treating umbilical cord blood stem cells with a serum-free medium supplemented with 5 types of growth factors and the exosomes isolated from the culture medium has a higher total protein content and extracellular matrix protein content, when compared to a culture medium produced by culturing umbilical cord blood stem cells in a serum-free medium to which the growth factor is not added. In addition, exosomes released by umbilical cord blood stem cells cultured in a serum-free medium containing five growth factors have a high concentration, a small size, and an even distribution.

The umbilical cord blood stem cell culture medium prepared according to the present invention contains a high content of high-potency exosomes and has a high skin regeneration and/or anti-inflammatory effect, so it can be used in a cosmetic composition for skin application requiring skin penetration and a pharmaceutical composition for wound treatment.

## Description

### Field of the Invention

The present invention relates to a method of preparing a culture medium in which high-potency exosomes are released in a high content by umbilical cord blood stem cells, and to a use thereof.

The umbilical cord blood stem cell culture medium prepared according to the present invention contains a high content of exosomes with high efficacy, which can be applied to and induce permeation into biological membranes such as skin. Therefore, it can be usefully used as a pharmaceutical composition, such as a wound treatment, and a cosmetic composition.

### Background of the Invention

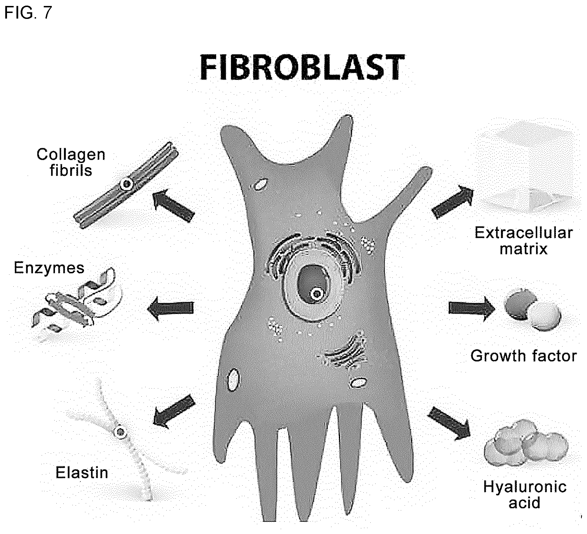

Mesenchymal stem cells release various growth factors such as epidermal growth factor and fibroblast growth factor and various cytokines. Therefore, it is known that they play an important role in skin regeneration by promoting the production of collagen from fibroblasts (FIG. 7). There is a lot of interest in the development of cosmetics using stem cells with these characteristics. In particular, research related to the development of a technology that can increase the penetration ability of the effective factors of stem cells into the skin is being conducted, and one of them is to utilize exosomes.

Exosomes are small vesicles with a membrane structure released from various types of cells. The diameter of the exosomes is reported to be approximately 30 ~ 150 nm. It was observed in studies through electron microscopy that exosomes originate in specific compartments within cells called multivesicular bodies (MVBs), rather than directly detach from the plasma membrane, and are released and secreted out of the cells. That is, when multivesicular body fuses with the plasma membrane, small vesicles are released into the extracellular environment, which is called exosomes. Although it is not clear what molecular mechanism these exosomes are made by, it is known that exosomes released from almost all living cells contain various components such as receptors, proteins and miRNAs and play an important role in intercellular signal transduction. In addition, the exosomes contain relatively little animal serum compared to stem cells, so the risk of zoonosis caused by animal serum infection can also be excluded. Considering these properties of exosomes, treatment using exosomes is expected to be a new paradigm that can overcome the limitations of existing stem cell therapies.

The skin is composed of the epidermis and dermis.

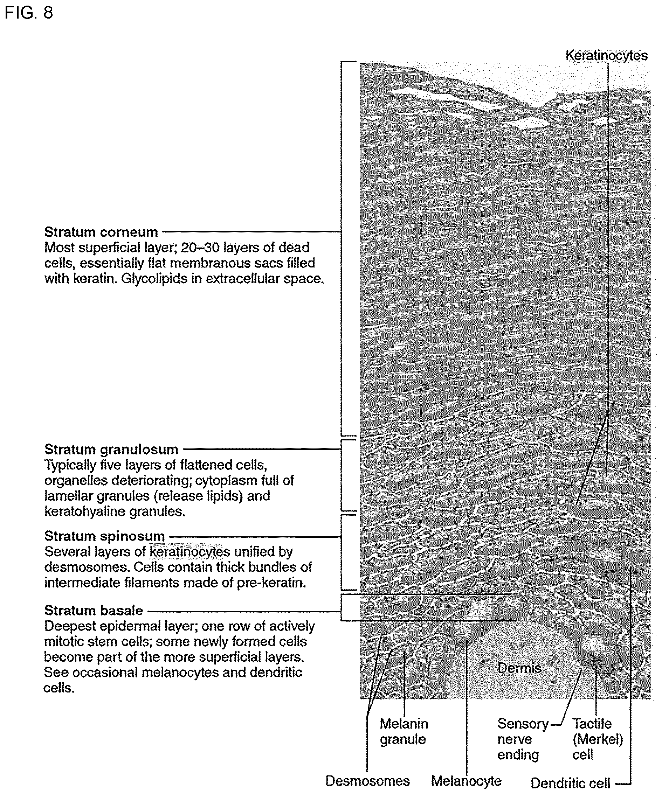

Cells that fill the epidermis include keratinocytes, melanocytes, dendritic cells and tactile cells. Most epidermal cells are keratinocytes (FIG. 8). The main function of keratinocytes is to form a barrier against environmental damage by heat, ultraviolet light, water loss, pathogenic bacteria, fungi, parasites and viruses. A number of structural proteins (filaggrin, keratin), enzymes (proteases), lipids and antimicrobial peptides (defensins) contribute to maintaining the important barrier function of the skin. Keratinogenesis is part of the physical barrier formation (keratinization), where keratinocytes produce more and more keratin and undergo terminal differentiation. The fully keratinized keratinocytes that form the outermost layer are continuously removed and replaced with new ones.

The dermis layer is 15 to 40 times thicker than the epidermis layer (0.04 mm to 1.6 mm), occupies most of the skin, and maintains the elasticity of the skin, and acts as a connective tissue between the epidermis and subcutaneous tissue. There are skin appendages, blood vessels, lymphatic vessels, muscles, nerves, etc., and the dermis layer is largely divided into two layers: the papillary dermis and the reticular dermis.

The papillary dermis occupies only a small portion of the total thickness of the dermis and is composed of collagen, blood vessels, and fibrocytes, and provides nutrients to the basal layer of the avascular epidermis. The reticular dermis is the layer that occupies most of the dermis, and consist of elastic fibers and various matrix proteins, where elastin and collagen fibers are arranged in thick bundles to give strength and flexibility to the skin. Collagen accounts for about 25% of the total protein in the connective tissue and plays an important role in the tensile strength of the dermal layer. Meanwhile, matrix metalloprotease (MMP) is a zinc-dependent endopeptidase that hydrolyzes the extracellular matrix (ECM). MMP decomposes ECM to increase cell mobility and circulates skin tissue, but forms wrinkles to promote skin aging and promote cancer cell metastasis. The formation of MMP is promoted by UV rays, stress, antibiotics, etc. Fibronectin is a glycoprotein present on the cell surface, connective tissue, and blood. Cells are not directly connected to collagen, but via fibronectin.

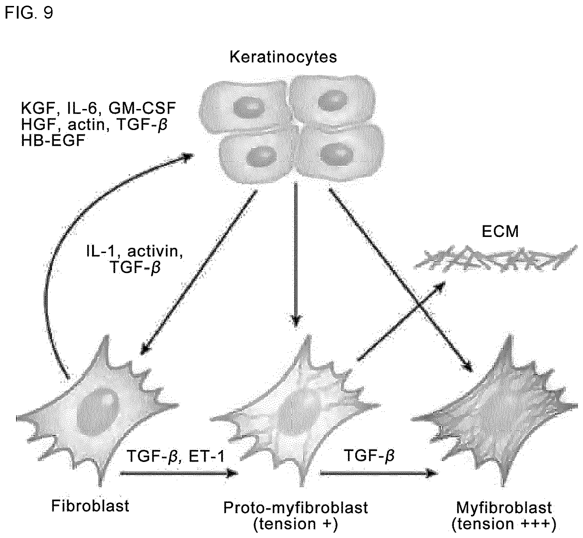

As shown in FIG. 9, fibroblasts produce and secrete both growth factors and cytokines required by keratinocytes. Keratinocytes are epidermal cells that are most easily exposed to the environment. Fibroblasts can be activated through keratinocytes, and through activation, the extracellular matrix is reformed to even out the skin layer.

There are bio-membranes (covering and lining membranes) as an upper concept of the skin. Covering and lining membranes are continuous multicellular sheets composed of epithelial tissue bound to an underlying layer of connective tissue. Bio-membrane (covering and lining membranes) include a skin membrane (Cutaneous Membrane), a mucous membrane, and a serous membrane.

As the aging population increases along with the improvement of living standards of modern people, interest and demand for functional cosmetics related to anti-aging, wrinkle improvement, whitening, and UV protection are increasing. However, since most basic cosmetics or functional cosmetics are manufactured with chemical substances, safety issues for the human body have been constantly raised. Accordingly, interest in products containing natural and organic raw materials is increasing, and the market size of related products is also increasing.

The development of products using these natural raw materials is actively progressing in the pharmaceutical field, forming the biopharmaceutical market. Biopharmaceuticals are a step forward from pharmaceuticals based on conventional chemical components. Biopharmaceutical raw materials are developed using cells, tissues, and hormones derived from humans or other living organisms. Existing chemical medicines have the disadvantage of having severe side effects and showing temporary improvement, but biopharmaceuticals have the advantage of fundamentally solving the cause of the disease without side effects and are currently being actively studied around the world. Accordingly, the present invention intends to utilize umbilical cord blood stem cell-derived exosomes as a main raw material in the next-generation biopharmaceutical field.

### BRIEF SUMMARY OF THE INVENTION

An object of the present invention is to provide a culture medium in which exosomes with high efficacy are released in a high content by umbilical cord blood stem cells, in order to increase the regeneration ability of biological membranes (covering and lining membranes) such as skin.

In the present invention, as a result of analyzing the culture medium obtained by growing umbilical cord blood stem cells in a serum-free medium supplemented with a specific growth factor cocktail containing two or more growth factors, it was found that the content of extracellular matrix protein was high. As a result of separating and analyzing the exosomes from the culture medium, it was found that the concentration of the active ingredient in the exosomes was high and the efficacy thereof was improved. In addition, it was found that when the high-potency exosomes of the present invention were treated with human fibroblasts and keratinocytes, the wound healing ability was improved through cell proliferation and migration ability. The present invention is based on these findings.

A first aspect of the present invention provides a method of producing a culture medium containing exosomes released by umbilical cord blood stem cells, comprising the step of culturing umbilical cord blood stem cells in a serum-free medium supplemented with one or two or more growth factors selected from the group consisting of GDF-11, EGF, FGF2, TGF-β1, and VEGF at a concentration of 1 ~ 20 ng/ml, respectively, to produce a culture medium containing exosomes with a released concentration of 0.5 ~ 5×10⁹/ml, preferably 1-3x109/ml during culturing of umbilical cord blood stem cells.

For example, umbilical cord blood stem cells can be cultured in a serum-free medium supplemented with GDF-11; and one or more growth factors selected from the group consisting of EGF, FGF2, TGF-β1, and VEGF at a concentration of 1 ~ 20 ng/ml, respectively.

Preferably, the umbilical cord blood stem cells can be cultured in a serum-free medium to which GDF-11, EGF, FGF2, TGF-β1, and VEGF are all added at a concentration of 1 to 20 ng/ml, respectively.

A second aspect of the present invention provides a composition for applying to skin or covering and lining membranes, comprising the exosome-containing culture medium obtained by the method according to the first aspect of the present invention or exosomes isolated from the culture medium, as an active ingredient.

A third aspect of the present invention provides a pharmaceutical composition for treating wounds or cuts, comprising the exosome-containing culture medium obtained by the method according to the first aspect of the present invention or exosomes isolated from the culture medium, as an active ingredient.

### BRIEF DESCRIPTION OF THE DRAWINGS/FIGURES

FIG. 1 is a result of comparative analysis of the total protein concentration and the extracellular matrix protein content present in the umbilical cord blood stem cell culture medium prepared for each condition according to Example 1.
FIG. 2 is a result of comparative analysis of the distribution, size, and concentration of exosomes present in the umbilical cord blood-derived stem cell culture medium prepared for each condition according to Example 1.
FIG. 3 is a result of comparative analysis of the effect of exosomes of umbilical cord blood-derived stem cell culture medium prepared for each condition according to Example 1 on the mobility of human fibroblasts and keratinocytes.
FIG. 4 is a result of comparative analysis of the effect of exosomes of umbilical cord blood-derived stem cell culture medium prepared for each condition according to Example 1 on the proliferative capacity of human fibroblasts and keratinocytes.

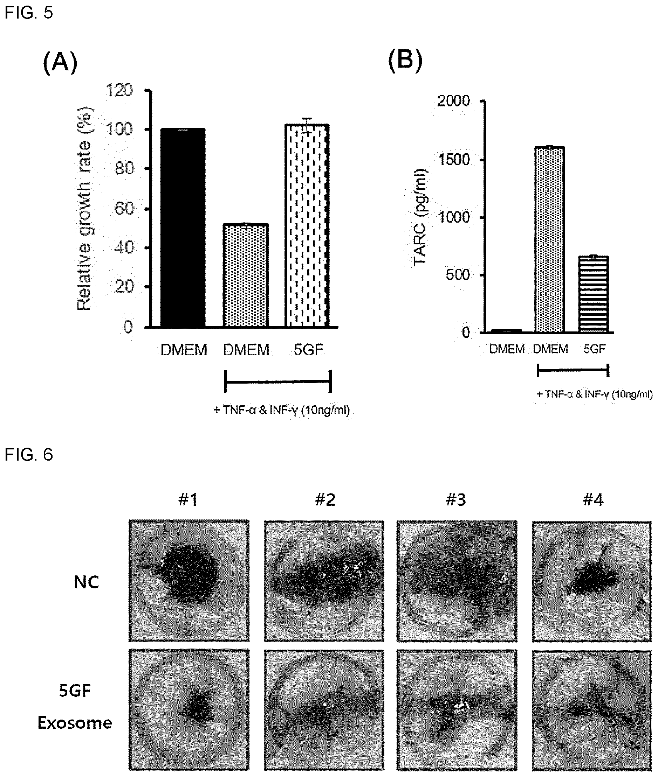

FIG. 5 is a result of comparative analysis of the effect on skin cell regeneration by treating the exosomes of the umbilical cord blood-derived stem cell culture medium prepared for each condition according to Example 1 to an animal wound model.
FIG. 6 is a conceptual diagram illustrating a biological membrane (covering and lining membranes), which is a superordinate concept of skin.
FIG. 7 is a conceptual diagram showing various active ingredients produced by fibroblasts.
FIG. 8 is a conceptual diagram showing the main structural features of the skin epidermis.
FIG. 9 is a conceptual diagram illustrating the interaction between fibroblasts and keratinocytes.

### DETAILED DESCRIPTION OF THE INVENTION

### 1. Stem Cell Culture Medium

Adult stem cells are cells in an undifferentiated state that differentiate into cells of a specific tissue when necessary. Adult stem cells may be mesenchymal stem cells, mesenchymal stromal cells, or pluripotent stem cells, but are not limited thereto. Unlike embryonic stem cells extracted from human embryos, adult stem cells have the advantage of avoiding ethical disputes because they are extracted from already grown body tissues such as bone marrow or brain cells. In the present invention, adult stem cells may be derived from umbilical cord, umbilical cord blood, bone marrow, fat, muscle, nerve, skin, amniotic membrane or placenta, but is not limited thereto.

Umbilical cord blood-derived stem cells use umbilical cord blood formed during the donor's gestational cycle (40 weeks), so there is an advantage that there is no difference in efficacy depending on the donor's condition, unlike adipose or bone marrow-derived adult stem cells.

The medium for culturing cells refers to a composition containing essential components necessary for the growth and proliferation of cells in vitro, and includes any medium commonly used in the art for culturing stem cells, for example, DMEM (Dulbecco's Modified Eagle's Medium), MEM (Minimal Essential Medium), BME (Basal Medium Eagle), RPMI 1640, DMEM/F-10 (Dulbecco's Modified Eagle's Medium: Nutrient Mixture F-10), DMEM/F-12 (Dulbecco's Modified Eagle's Medium) : Nutrient Mixture F-12), α-MEM (α-Minimal essential Medium), G-MEM (Glasgow's Minimal Essential Medium), IMDM (Isocove's Modified Dulbecco's Medium), KnockOut DMEM, etc., but is not limited thereto. A commercially prepared medium or an artificially synthesized medium may be used. The medium of the present invention generally includes a carbon source, a nitrogen source and a trace element component, and may further include various growth factors as well as amino acids and antibiotics.

In the present invention, growth factors such as GDF-11, EGF, FGF2, TGF-β1, and VEGF can be added to the medium for culturing cells and/or produced by the umbilical cord blood stem cells.

Growth factor refers to a proteinaceous physiologically active substance that promotes cell division, growth, and differentiation, for example, GDF-11 (Growth and differentiation factor 11), brain-derived neurotropic factor (BDNF), fibroblast growth factor (FGF), hepatocyte growth factor (HGF), nerve growth factor (NGF), vascular epithelial growth factor (VEGF), Insulin-like growth factor (IGF), transforming growth factor (TGF), platelet-derived growth factor (PDGF), bone-derived growth factor (BDF), colony stimulation factor (CSF), epidermal growth factor (EGF), keratinocyte growth factor (KGF), and the like.

As confirmed in Experimental Examples 1 to 5, according to the present invention, GDF-11, EGF, FGF2, TGF-β1, and VEGF added to the serum-free medium are interpreted to stimulate signaling pathways of receptors on umbilical cord blood stem cells corresponding to respective growth factors, and to promote the SMAD signaling pathway and the like. As a result, it is interpreted that the synthesis of collagen and fibronectin protein is increased, and the contents of growth factors in the exosomes, collagen, and fibronectin are increased. It is interpreted that the increased collagen, fibronectin, and growth factor-containing exosomes affect the proliferation and migration ability of human fibroblasts and keratinocytes. In addition, due to the autocrine effect and paracrine effect, the re-secretion of EGF, FGF2, TGF-β1, VEGF, and/or GDF-11 is increased, and the re-secretion of other growth factors is promoted, which affects the proliferation and migration ability of surrounding cells.

Therefore, in the present invention, the culture medium may refer to the supernatant of the cell culture medium after culturing the umbilical cord blood stem cells. The umbilical cord blood stem cell culture medium contains various physiologically active substances secreted from the cells during the culturing of the umbilical cord blood stem cells. The physiologically active substance is a generic term for cytokines, cell growth factors, immunomodulatory factors, etc. that can affect cells or body functions, and examples of the physiologically active substances include VEGF (vascular endothelial growth factor), EGF(epidermal growth factor), HGF(hepatocyte growth factor), TGF-beta(tumor growth factor-beta), and IGF(insulin growth factor), but are not limited thereto.

### 2. Exosomes released by stem cells

Exosomes are evaluated as ideal vesicles for drug delivery because they can deliver substances across the cell membrane. Since such a lipid bilayer vesicle system can overcome the skin penetration problem, it is regarded as one of the most effective strategies for delivering drugs to the dermal layer of the skin(Saahil Arora et al., Asian Journal of Pharmaceutics, 6, 4, 237-244, 2012).

Exosomes contain RNAs, proteins, lipids and metabolites that reflect the cell types from which the exosomes are derived. Exosomes contain various molecular components (e.g., proteins and RNAs) of the cell from which the exosomes are derived. The protein composition in exosomes varies depending on the cell and tissue from which the exosomes are derived, but most exosomes contain a common set of evolutionarily conserved protein molecules.

Exosomes can be obtained by separating from the culture medium obtained after culturing cells, and the size and content of exosomes can be affected by molecular signals received by cells producing exosomes.

In the present invention, the culture medium itself can be used as a raw material without separating the exosomes, but the exosomes can be separated and used. In addition, the exosomes can be separated to confirm the size and physical properties of the exosomes in the culture medium for quality assurance and the like.

As a method for isolating the exosomes from the culture medium, centrifugation method, immunobinding method, filtration method, etc. can be used.

Ultracentrifugation, the most commonly used exosome separation method, cannot separate a large amount of exosomes at once, requires expensive equipment, takes a lot of time to separate, and physical damage to exosomes may occur due to strong centrifugation and in particular, there are disadvantages such as a decrease in the purity of the isolated exosomes. Among the methods for improving this disadvantage, the PS affinity method that increases the purity of the separated exosome by using a substance that specifically binds to phosphatidylserine (PS) present in the membrane of the exosome. is available. This can separate exosomes of high purity compared to the ultracentrifugation method, but has a disadvantage in that the yield is low.

The exosomes in the culture medium of umbilical cord blood mesenchymal stem cells (UCB-MSC) contain various growth factors such as EGF, VEGF, TGF, HGF, FGF, IGF and PDGF at a higher level than the exosomes in the culture medium of adipose tissue-derived mesenchymal stem cells or bone marrow-derived mesenchymal stem cells. Growth factors such as EGF promote the proliferation of fibroblasts, which are constituent cells of the skin, promote cell migration and collagen synthesis, so the UCB-MSC-derived exosomes can exhibit excellent skin condition improvement effects and wound healing effects such as skin regeneration, skin elasticity improvement, skin wrinkle prevention or improvement, skin aging prevention or improvement, hair growth or restoration of reduced hair follicles.

The culture medium of stem cells prepared according to the present invention not only contains a large amount of exosomes, but also provides a large amount of nano-sized exosomes that can penetrate to the dermal layer of the skin and enhance the regeneration effect due to the same lipid bilayer structure as the cell membrane. Since they contain a large amount of growth factors within the exosomes, exosomes can exert skin regeneration and anti-aging effect, increase in collagen synthesis, hair growth, restoration of reduced hair follicles and wound healing effect through the proliferation and activation of the fibroblast, which are the skin cells.

The exosomes of the present invention may be produced or used as a culture medium containing them or may be produced or used in a state in which cells are removed from the culture medium. Since the exosome-containing culture medium from which cells are removed is a cell-free formulation, there is little risk of cancer and no problem of transplant rejection, and there is no risk of occlusion of microvessels when administered systemically. Because it is a cell-free formulation, it is possible to develop drugs as off-the-shelf products, thereby reducing the manufacturing cost.

The feature of the present invention is to culture umbilical cord blood stem cells in a serum-free medium supplemented with EGF, FGF2, TGF-β1, VEGF and/or GDF-11 at a concentration of 1 ~ 20 ng/ml, respectively in order to produce a culture medium having a high concentration of exosomes at 0.5~5×10⁹/ml, preferably 1~3×10⁹/ml, released during culturing of umbilical cord blood-derived stem cells.

As a result of culturing umbilical cord blood mesenchymal stem cells under specific culture conditions in which EGF, FGF2, TGF-β1, VEGF, and/or GDF-11, which are growth factors, were added at a concentration of 1 to 20 ng/ml, respectively, according to the present invention, it was found that the culture medium contains a higher concentration (1~3×10⁹/ml) of exosomes, when compared to a culture medium produced by culturing umbilical cord blood stem cells in a serum-free medium to which the growth factor is not added, and such exosomes of the present invention improve the migration and proliferation ability of human fibroblasts and human keratinocytes. In addition, it was found that the culture medium produced by culturing umbilical cord blood stem cells in a serum-free medium supplemented with EGF, FGF2, TGF-β1, VEGF, and/or GDF-11 at a concentration of 1 to 20 ng/ml, respectively contained a large amount of fibronectin protein and/or collagen protein, which is an extracellular matrix. Furthermore, it was found to produce a large amount of exosomes with a small particle size of 100 nm ± 20 nm in diameter that are easy to permeate through the skin. The present invention is based on these findings.

The culture medium produced when culturing umbilical cord blood stem cells according to the present invention contains a large amount of exosomes containing a high concentration of a growth factor, secreted exosomes having a uniform size at a high concentration. The culture medium of the present invention or exosomes isolated from the culture medium may be an active ingredient of a composition for applying to biological membranes (i.e., covering and lining membranes) such as skin.

The present invention can mass produce exosomes with a size of about 120 nm that penetrate the skin, through cell culture of umbilical cord blood stem cells. In addition, the cell culture medium obtained after culturing the umbilical cord blood stem cells according to the present invention has a high content of exosomes that can improve the mobility and proliferation of human fibroblasts, has a high permeability of biological membranes (covering and lining membranes) such as skin in culture medium, and contains a large amount of fibronectin protein and/or collagen protein. Therefore, it is a preferable active ingredient of a formulation (e.g., pharmaceutical composition, cosmetics, quasi-drugs) for applying to biological membranes (i.e., covering and lining membranes) such as skin, or a preferable active ingredient of pharmaceutical composition for treating to wounds or cuts, and it can exhibit excellent efficacy when used.

### 3. Various formulations

The exosome-containing culture medium obtained by the umbilical cord blood-derived stem cells according to the present invention and/or the exosomes isolated from the culture medium can be formulated in various ways according to the purpose of use.

For example, the exosome-containing culture medium or exosomes isolated from the culture medium is used as it is in the liquid phase; is gelled; or is frozen and/or dried to powder.

It can be mixed with components such as hydrogel, gelatin, etc., and then be used in a composition for applying to skin or biomembrane (covering and lining membranes), which can control gelation and solation due to the difference in viscosity according to the content ratio of such components. By mixing with components such as poloxamer, the formulation can be designed so that it gels at 4°C and solubilizes at body temperature.

Cosmetic compositions may be prepared in a formulation selected from the group consisting of solutions, external ointments, creams, foams, nutritional lotions, softened lotions, perfumes, packs, soft water, makeup bases, essences, soaps, liquid detergents, bath agents, sunscreen creams, sun oils, suspensions, emulsions, paste, gel, lotion, powder, soap, surfactant-containing cleansing, oil, powder foundation, emulsion foundation, wax foundation, patch and spray, but are not limited thereto.

The cosmetic composition may further include one or more cosmetically acceptable carriers to be formulated in general skin cosmetics, and conventional ingredients such as oil, water, surfactant, humectant, lower alcohol, thickener, chelating agent, pigment, preservatives, fragrances, etc. may be appropriately blended, but the present invention is not limited thereto.

The cosmetically acceptable carrier included in the cosmetic composition varies depending on the formulation of the cosmetic composition.

When the formulation is an ointment, paste, cream or gel, animal oil, vegetable oil, wax, paraffin, starch, tragacanth, cellulose derivative, polyethylene glycol, silicone, bentonite, silica, talc, zinc oxide, etc., but is not limited thereto, may be used as a carrier component. These components may be used alone or in combination of two or more.

When the formulation is a powder or a spray, lactose, talc, silica, aluminum hydroxide, calcium silicate, polyamide powder, etc., but is not limited thereto, may be used as a carrier component. Especially in the case of a spray, it may additionally contain a propellant such as chlorofluorohydrocarbon, propane/butane or dimethyl ether, but is not limited thereto. These components may be used alone or in combination of two or more.

When the formulation is a solution or emulsion, a solvent, solubilizer or emulsifier may be used as a carrier component. For example, water, glycerin, ethanol, isopropanol, ethyl carbonate, ethyl acetate, benzyl alcohol, benzyl benzoate, propylene glycol, or 1,3-butylglycol oil, etc., but is not limited thereto, can be used and especially, cottonseed oil, peanut oil, corn germ oil, olive oil, castor oil, sesame oil, glycerol aliphatic ester, fatty acid ester of polyethylene glycol or sorbitan may be used. These may be used alone or in combination of two or more.

When the formulation is a suspension, a liquid diluent such as water, glycerin, ethanol or propylene glycol, a suspending agent such as ethoxylated isostearyl alcohol, polyoxyethylene sorbitol esters and polyoxyethylene sorbitan esters, microcrystalline cellulose, aluminum metahydroxide, bentonite, agar or tracanth and the like, but is not limited thereto, can be used as the carrier component. These may be used alone or in combination of two or more.

When the formulation is a soap, alkali metal salts of fatty acids, fatty acid hemiester salts, fatty acid protein hydrolysates, isethionate, lanolin derivatives, aliphatic alcohols, vegetable oils, glycerol, sugar, etc., but is not limited thereto, may be used as carrier components. These may be used alone or in combination of two or more.

In the cosmetic composition, the exosome may be included in an amount of 0.0001 to 50% by weight of the total weight of the cosmetic composition, and more specifically, it may be included in an amount of 0.0005 to 10% by weight. When the exosome is included within the above range, there is an advantage of exhibiting an excellent skin condition improvement effect, and there is an advantage that the formulation of the composition is stabilized.

Among the items used for the purpose of diagnosing, treating, ameliorating, alleviating, treating or preventing diseases of humans or animals, generally, quasi-drugs refer to those products that have a milder action than drugs or products, excluding those used for pharmaceutical purposes. It includes products used for the treatment or prevention of diseases in humans or animals, and products with minor or no direct action on the human body.

The quasi-drug composition may be prepared in a formulation selected from the group consisting of body cleanser, foam, soap, mask, ointment, cream, lotion, essence, and spray, but is not limited thereto.

When the present invention is a composition for applying to covering and lining membranes such as skin, or a pharmaceutical composition for treating to wounds or cuts, a pharmaceutically acceptable carrier can be included.

"Pharmaceutically acceptable" means that it can be used routinely in the field of pharmaceuticals, which does not stimulate the organism upon administration and does not impair the biological activity and properties of the administered compound.

The dosage may be a pharmaceutically effective amount for improving skin condition. The term "pharmaceutically effective amount" refers to an amount sufficient to treat a disease with a reasonable benefit/risk ratio applicable to medical treatment, and the effective dose level may depend on factors, including the subject type and severity, age, sex, type of disease, drug activity, sensitivity to drugs, administration time, administration route and excretion rate, duration of treatment, concomitant drugs, and other factors well known in the medical field. In addition, effective amounts may vary depending on the route of treatment, the use of excipients, and the potential for use with other agents, as will be appreciated by those skilled in the art.

The type of carrier is not particularly limited, and any carrier commonly used in the art may be used. Although not limited thereto, examples include saline, sterile water, Ringer's solution, buffered saline, albumin injection solution, lactose, dextrose, sucrose, sorbitol, mannitol, xylitol, erythritol, maltitol, maltodextrin, glycerol, ethanol, and the like. These may be used alone or in combination of two or more.

If necessary, other pharmaceutically acceptable additives such as excipients, diluents, antioxidants, buffers or bacteriostats may be added and used, and fillers, extenders, wetting agents, disintegrants, dispersants, surfactants, binders or lubricants may be added.

The umbilical cord blood mesenchymal stem cell culture medium prepared according to the present invention contains a high content of high-potency exosomes having a uniform size distribution, and can be usefully used in a cosmetic composition requiring skin penetration and a pharmaceutical composition for treating to wounds or cuts.

Hereinafter, the present invention will be described in more detail through examples. These examples are for illustrative purposes only, and it will be apparent to those of ordinary skill in the art that the scope of the present invention is not construed as being limited by these examples.

### Example 1. Preparation of umbilical cord blood stem cell culture medium and extraction of exosomes released by umbilical cord blood stem cells

Humam umbilical cord blood stem cells were cultured in KSB-3 (Basal Culture Medium) containing 10% fetal bovine serum in a 5% CO₂ incubator at 37°C for 1 to 4 days, followed by removing the culture medium, and they were washed with PBS.

DMEM medium containing GDF-11 (1-20 ng/ml), EGF (1-20 ng/ml), FGF2 (1-20 ng/ml), TGF-β1 (1-20 ng/ml) and VEGF (1-20 ng/ml) and DMEM medium without such growth factors were added to the washed cells, respectively, prior to culturing for 1 to 4 days at 37° C, in a 5% CO₂ incubator, and then centrifuging at 500 g for 5 minutes. After separation, the supernatant was separated and filtered through a 0.2 µm-0.8 µm filter.

Exosomes were extracted from the culture medium produced under each condition and filtered through a 0.2 µm ~ 0.8 µm filter using Capturem^{™} Exosome Isolation Kit (Takara Bio). The extracted exosomes were used for each test after buffer exchange with PBS using Amicon Ultra Centrifugal Filter (Merck Millipore).

### Experimental Example 1. Analysis of extracellular matrix protein content in umbilical cord blood stem cell culture medium

The extracellular matrix in the connective tissue, which is responsible for structural support of cells, typically contains components such as fibronectin, collagen, and elastin, and plays a role in regenerating damaged tissues.

BCAAssay was performed to analyze the total amount of proteins in the umbilical cord blood stem cell culture medium, and an enzyme-linked immunoprecipitation assay (ELISA assay) was performed to analyze the extracellular matrix protein content in the umbilical cord blood stem cell culture medium. The culture medium of each condition prepared in Example 1 was comparatively analyzed with Human Fibronectin Quantikine ELISA Kit (R&D System, MN, USC) and Procollagen Type I C-peptide (PIP) EIA Kit (Takara bio inc, Japan).

Concretely, the total protein content and the extracellular matrix protein component content in the culture medium (DMEM) prepared by using a medium not containing a growth factor and the culture medium (5GF) prepared by using a medium added GDF-11, EGF, FGF2, TGF-β1, and VEGF at a concentration of 1 ~ 20ng / ml, respectively, were compared and analyzed.

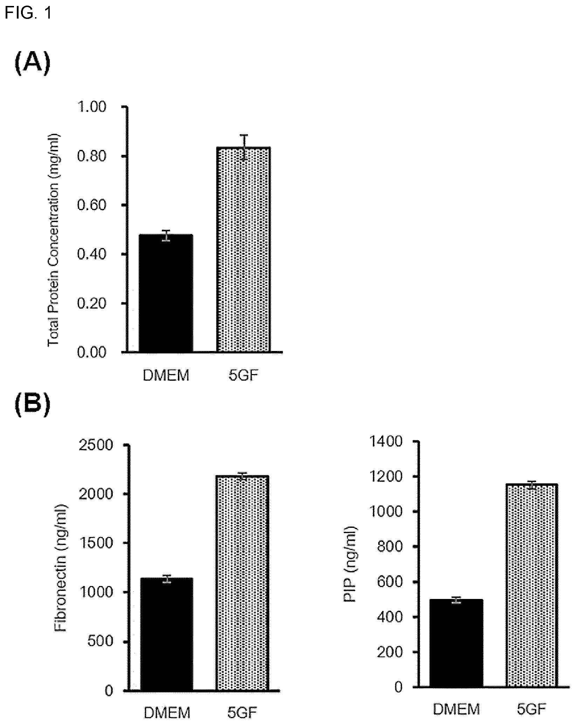

As a result, as shown in FIG. 1A, it was confirmed that the total amount of protein and fibronectin content present in the culture medium (5GF) prepared by using a medium to which GDF-11, EGF, FGF2, TGF-β1, and VEGF were added at a concentration of 1 to 20 ng/ml, respectively were higher than those of the control (DMEM). In addition, as shown in FIG. 1B, it was confirmed that the content of collagen (PIP) in the culture medium (5GF) was higher than that of the control group (DMEM).

### Experimental Example 2. Analysis of the size and concentration of exosomes present in the umbilical cord blood stem cell culture medium

After separating the exosomes from the umbilical cord blood stem cell culture medium prepared by culturing for each condition in Example 1, the size and concentration of the exosomes present in the culture medium were compared and analyzed using Nanosight. Nanosight can analyze the distribution and concentration of nanoparticle size by observing and analyzing individual particles that are diffusing in size from 10nm to 2000nm in solution.

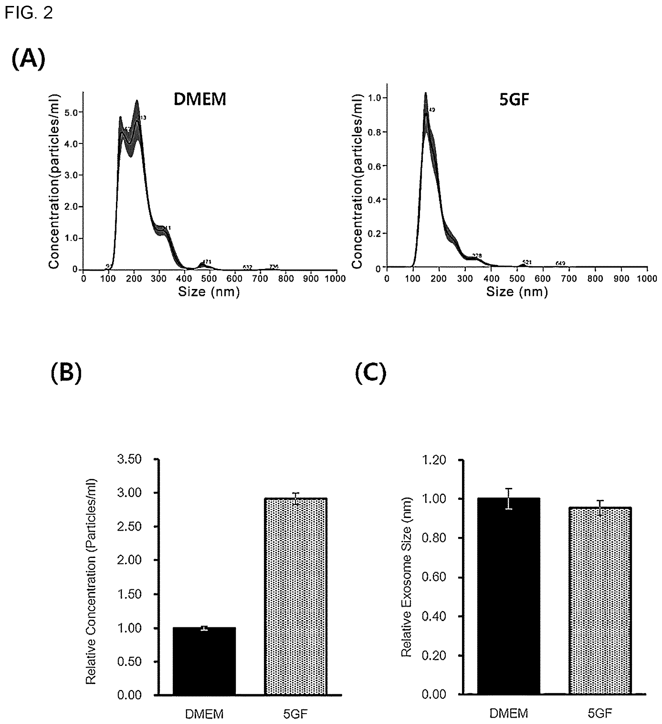

As a result, as shown in FIG. 2, when prepared by using a medium supplemented with GDF-11, EGF, FGF2, TGF-β1, and VEGF at a concentration of 1 to 20 ng/ml (5GF), it was confirmed that the number of exosomes was higher, compared to the control group (DMEM) using a medium not containing such growth factors and the size distribution was uniformly around 120 nm.

### Experimental Example 3. Effect of exosomes in umbilical cord blood stem cell culture medium on the migration ability of human fibroblasts and keratinocytes

The migration ability of epidermal cells is a major mechanism in wound healing. To determine the effect of exosomes in the culture medium of umbilical cord blood stem cells on the migration ability of human dermal fibroblasts (HDF) and keratinocytes (HKC), a Trans-well migration assay was performed.

Human fibroblasts and keratinocytes were each aliquoted into the trans-well insert at 2×10⁴ cells/well, cultured for one day at 37°C under 5% CO₂ condition, the culture medium was removed, and then washed with PBS. 100ul/well of Serum-free DMEM containing exosomes prepared and extracted in Example 1 for each condition was treated, and 2 hours later, 400ul of DMEM containing 10% serum was added to the bottom plate and cultured at 37°C 5% CO₂ condition for one day. After staining with Cristal Violet Solution to stain the migrated cells, they were comparatively analyzed with an optical microscope.

Concretely, 2×10⁸ exosomes extracted from a culture medium prepared by using a growth factor-free medium (DMEM) and a medium (5GF) containing GDF-11, EGF, FGF2, TGF-β1, and VEGF added at a concentration of 1 to 20 ng/ml, respectively, were treated into human fibroblasts. A basal medium (Basal DMEM) in which umbilical cord blood stem cells were not cultured was used as a negative control (non treat).

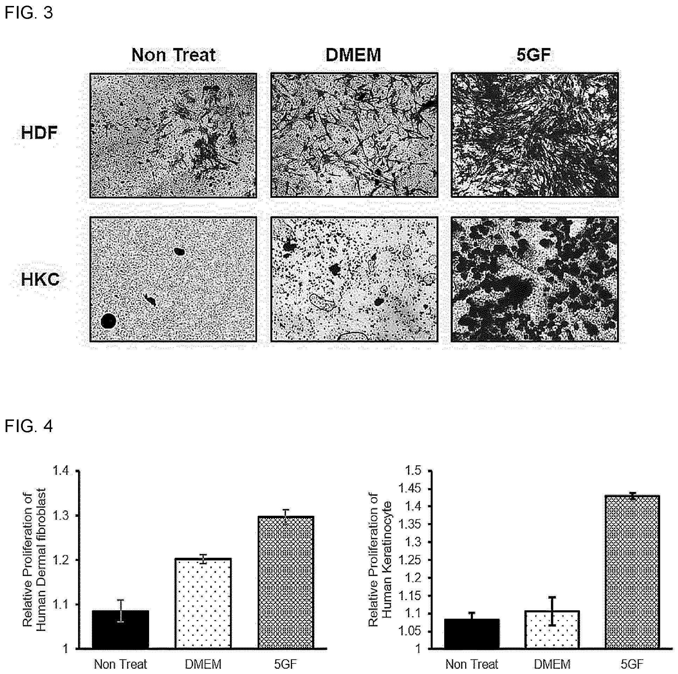

As a result, as can be seen in Figure 3, it was confirmed that the migration ability of human fibroblasts and keratinocytes treated with exosomes isolated from a culture medium of umbilical cord blood stem cells prepared by using a medium supplemented with GDF-11, EGF, FGF2, TGF-β1, and VEGF at a concentration of 1 to 20 ng/ml, respectively was increased compared to the control groups.

### Experimental Example 4. Effect of exosomes in umbilical cord blood stem cell culture medium on the proliferative capacity of human fibroblasts and keratinocytes

In healing wounds, proliferation of fibroblasts and keratinocytes is also an important part of the regeneration process. In order to confirm the effect of the culture medium on the proliferation of human fibroblasts and keratinocytes, a Proliferation Assay was performed. Human fibroblasts and keratinocytes were each dispensed at 1×10⁵ cells/well in a 6-well plate and cultured at 37°C under 5% CO₂ conditions. The number of cells was measured at 24 hour intervals.

Concretely, 2×10⁸ exosomes extracted from a culture medium prepared by using a growth factor-free medium (DMEM) and a medium (5GF) containing GDF-11, EGF, FGF2, TGF-β1, and VEGF added at a concentration of 1 to 20 ng/ml, respectively, were treated into human fibroblasts and keratinocytes. A basal medium (Basal DMEM) in which umbilical cord blood stem cells were not cultured was used as a negative control (non treat).

As a result, as can be seen in Figure 4, it was confirmed that the proliferative capacity of human fibroblasts and keratinocytes treated with exosomes isolated from a culture medium of umbilical cord blood stem cells prepared by using a medium supplemented with GDF-11, EGF, FGF2, TGF-β1, and VEGF at a concentration of 1 to 20 ng/ml, respectively was increased compared to the control groups.

### Experimental Example 5. Effect of exosomes in umbilical cord blood stem cell culture on skin tissue regeneration in an animal wound model

As confirmed in vitro, in order to confirm whether exosomes released by umbilical cord blood stem cells improve the proliferation and migration ability of human skin cells in the same way, in vivo (in-vivo), the animal wound model test was conducted. A 15mm size wound was formed by punching on the back of a 6-week-old SD Rat, and then 2×10⁸ exosomes were treated. After 14 days, the skin tissue regeneration effect was confirmed.

Concretely, 2×10⁸ exosomes extracted from a culture medium prepared by using a medium (5GF) containing GDF-11, EGF, FGF2, TGF-β1, and VEGF added at a concentration of 1 to 20 ng/ml, respectively, were treated into the animal wound model. An animal wound model without any treatment was used as a negative control (NC).

As a result, as can be seen in FIG 4, it was confirmed that the skin tissue regeneration ability of the animal wound model treated with exosomes isolated from a culture medium of umbilical cord blood stem cells prepared by using a medium supplemented with GDF-11, EGF, FGF2, TGF-β1, and VEGF at a concentration of 1 to 20 ng/ml, respectively was significantly superior to that of the control group.

## Claims

1. A method of producing a culture medium containing exosomes released by umbilical cord blood stem cells, comprising
the step of culturing umbilical cord blood stem cells in a serum-free medium supplemented with one or two or more growth factors selected from the group consisting of GDF-11, EGF, FGF2, TGF-β1, and VEGF at a concentration of 1 ~ 20 ng/ml, respectively, to produce a culture medium containing exosomes with a released concentration of 0.5 ~ 5×10⁹/ml during culturing of umbilical cord blood stem cells.

2. The method of claim 1, wherein umbilical cord blood stem cells are cultured in a serum-free medium supplemented with GDF-11; and one or more growth factors selected from the group consisting of EGF, FGF2, TGF-β1, and VEGF at a concentration of 1 ~ 20 ng/ml, respectively.

3. The method of claim 1, wherein umbilical cord blood stem cells are cultured in a serum-free medium supplemented with GDF-11, EGF, FGF2, TGF-β1, and VEGF at a concentration of 1 ~ 20 ng/ml, respectively.

4. The method of claim 1, wherein the culture medium containing exosomes released by umbilical cord blood stem cells in the growth factor-added serum-free medium has a higher level of extracellular matrix protein fibronectin and/or collagen content, when compared to a culture medium produced by culturing umbilical cord blood stem cells in a serum-free medium to which the growth factor is not added.

5. The method of claim 1, wherein the culture medium containing exosomes released by umbilical cord blood stem cells in the growth factor-added serum-free medium has a greater number of released exosomes, contains a higher concentration of active ingredients in the exosomes, and has a higher ability to regenerate the skin and/or to inhibit inflammation, when compared to a culture medium produced by culturing umbilical cord blood stem cells in a serum-free medium to which the growth factor is not added.

6. The method of claim 1, wherein exosomes with a small particle size of 120 nm ± 20 nm in diameter, which are easily permeable through the skin, are mass-produced.

7. The method of claim 1, wherein the serum-free medium is at least one selected from the group consisting of DMEM (Dulbecco's Modified Eagle's Medium), MEM (Minimal Essential Medium), BME (Basal Medium Eagle), RPMI 1640, DMEM/F-10 (Dulbecco's Modified Eagle's Medium: Nutrient Mixture F-10), DMEM/F-12 (Dulbecco's Modified Eagle's Medium: Nutrient Mixture F-12), α-MEM(α-Minimal essential Medium), G-MEM(Glasgow's Minimal Essential Medium), IMDM (Isocove's Modified Dulbecco's Medium), and KnockOut DMEM.

8. The method of claim 1, further comprising the step of formulating the culture medium containing exosomes released by umbilical cord blood stem cells or the exosome isolated from the culture medium according to the purpose of use.

9. The method of claim 8, wherein the exosome-containing culture medium or exosomes isolated from the culture medium is used as it is in the liquid phase; is gelled; or is frozen or dried to powder.

10. The method of claim 1, wherein the exosomes released during culturing of umbilical cord blood stem cells in the growth factor-added serum-free medium improve the migration and/or proliferation ability of human fibroblasts and/or human keratinocytes, compared to exosomes released during culturing umbilical cord blood stem cells in a serum-free medium to which the growth factor is not added.

11. A composition for applying to skin or covering and lining membranes, comprising the exosome-containing culture medium obtained by the method according to any one of claims 1 to 10 or exosomes isolated from the culture medium, as an active ingredient.

12. The composition of claim 11, which is a cosmetic composition, a quasi-drug composition, or a pharmaceutical composition.

13. The composition of claim 11, wherein small exosomes from umbilical cord blood stem cells are used as transdermal carriers.

14. The composition of claim 11, which is a formulation gelled by mixing with a polymer, wherein the gelled formulation solitates under body temperature conditions.

15. A pharmaceutical composition for treating wounds or cuts, comprising the exosome-containing culture medium obtained by the method according to any one of claims 1 to 10 or exosomes isolated from the culture medium, as an active ingredient.
